# EUROPEAN PATENT SPECIFICATION

(11) **EP 4 223 442 B1**
(45) Date of publication and mention of the grant of the patent: **20.05.2026**
(21) Application number: 22217061.5
(22) Date of filing: 29.12.2022
(51) Int. Cl.: B23K 1/00, B23K 1/06, B23K 3/08, A61B 5/00, H01L 21/607, B23K 26/08, B23K 26/362

(54) **SYSTEM AND METHOD FOR SOLDERING LEADS TO PADS IN PRODUCING BASKET CATHETER**
SYSTEM UND VERFAHREN ZUM LÖTEN VON LEITUNGEN AN PADS BEI DER HERSTELLUNG EINES KORBKATHETERS
SYSTÈME ET MÉTHODE DE BRASURE DE CONDUCTEURS SUR DES COUSSINETS DANS LA PRODUCTION D'UN CATHÉTER À PANIER

(30) Priority: 30.12.2021 US 202117566400
(43) Date of publication of application: 09.08.2023
(73) Proprietor: Biosense Webster (Israel) Ltd, 2066717 Yokneam (IL)
(72) Inventor: GOVARI, Assaf, Yokneam 2066717 (IL); BEECKLER, Christopher Thomas, Irvine 92618 (US); KEYES, Joseph Thomas, Irvine 92618 (US); PAPAIOANNOU, Athanassios, Irvine 92618 (US); HERRERA, Kevin Justin, Irvine 92618 (US)
(74) Representative: Carpmaels & Ransford LLP

(56) References cited:
- US-A1- 2005 205 539
- US-A1- 2019 224 447

## Description

The present invention relates generally to medical devices, and particularly to a system and a method for producing basket catheters, see claims 1 and 5.

### BACKGROUND

Various techniques for welding and/or soldering leads in expandable catheters have been published. Some of these techniques use one or more lasers in the production process.

US 2019/0224447 A1 discloses a method of manufacturing a flexible catheter with a locatable sensor, including simultaneously rotating first and second spools about a common axis, whereby first and second leads of a wire twist together to form a twisted pair of the wire, and rotating a catheter body about a longitudinal axis defined by the catheter body, whereby the twisted pair of the wire wraps about a proximal end portion of the catheter body.

According to a first aspect of the present invention, a system is defined in claim 1.

Embodiments of the first aspect of the present invention are defined in its dependent claims.

According to a second aspect of the present invention, a method is defined in claim 5.

Further embodiments of the second aspect of the present invention are defined in its dependent claims.

The present invention will be more fully understood from the following detailed description of the examples thereof, taken together with the drawings in which:

### BRIEF DESCRIPTION OF THE DRAWINGS

Fig. 1 is a schematic, pictorial illustration of a catheter-based position-tracking and ablation system;
Fig. 2A is a schematic, pictorial illustration of splines of a catheter of the system presented in Fig. 1;
Fig. 2B is a schematic, pictorial illustration of a spline of the catheter;
Fig. 3 is a schematic, pictorial illustrations of a system for soldering a lead to a soldering pad of a spline; and
Fig. 4 is a flow chart that schematically illustrates a method for soldering a lead to a soldering pad of a spline.

### DETAILED DESCRIPTION OF EXAMPLES

### OVERVIEW

Expandable catheters, such as basket catheters, typically have electrical connections disposed in their splines for exchanging signals between the proximal and distal ends of the catheter. The distal end of a basket catheter may comprise over 100 connections (e.g., over about 10 connections for each spline), each connection comprises a pair of a lead and a solder pad that are soldered (or attached using another technique) to one another. The lead may have a diameter of about 32 µm, corresponding to American Wire Gauge (AWG) 48, or any other suitable shape and size, and soldering over ten pairs of the lead and respective solder pads within the footprint of a spline is a difficult and time-consuming task, which may also result in an electrical short between two or more adjacent connections.

Examples of the present invention that are described hereinbelow provide techniques for soldering leads to respective solder pads using improved soldering system and method.

The system for soldering comprises a soldering fixture, which is configured to hold (i) a substrate of a distal-end assembly (e.g., one or more splines) of a basket catheter and (ii) a lead, which is placed on a given solder pad disposed on the substrate.

The solder pads are arranged along the spline so as to increase the distance between any adjacent pads, and therefore, to simplify the soldering process and reducing the odds of obtaining undesired electrical shorts therebetween. In the present example, the solder pads are disposed along a virtual diagonal extending along the longitudinal axis of the spline.

The system comprises a laser assembly, which is configured to emit a laser beam, and to direct the laser beam toward the substrate of the spline.

The system comprises a positioning assembly, which is configured to move the soldering fixture, which holds the substrate and the lead, relative to the laser assembly, so as to mark on the lead and/or the given solder pad a soldering position, at which the lead is to be attached to the given solder pad, with a laser spot of the laser beam.

The system comprises a processor, which is configured to receive an image of the substrate of the spline having the plurality of solder pads disposed thereon. The processor is configured to apply a pattern recognition software for identifying: (i) the given solder pad from among the solder pads of the spline, and (ii) the lead positioned over the given solder pad.

The system comprises a soldering gun, which is configured to attached (e.g., solder) the lead to the given soldering pad at the soldering position. The soldering gun can comprise an ultrasound transducer, which is configured to ultrasonically attach the lead to the given soldering pad at the soldering position by applying an ultrasound signal to the soldering position. In other examples, the soldering gun may use any other soldering technique to carry out the soldering between the lead and the given solder pad.

The disclosed techniques improve the quality of basket catheters splines, and improve the productivity and automation of such soldering processes. The disclosed techniques may be used, *mutatis mutandis,* for producing other sorts of catheters, and for soldering leads to respective pads in other sorts of electronic systems.

### SYSTEM DESCRIPTION

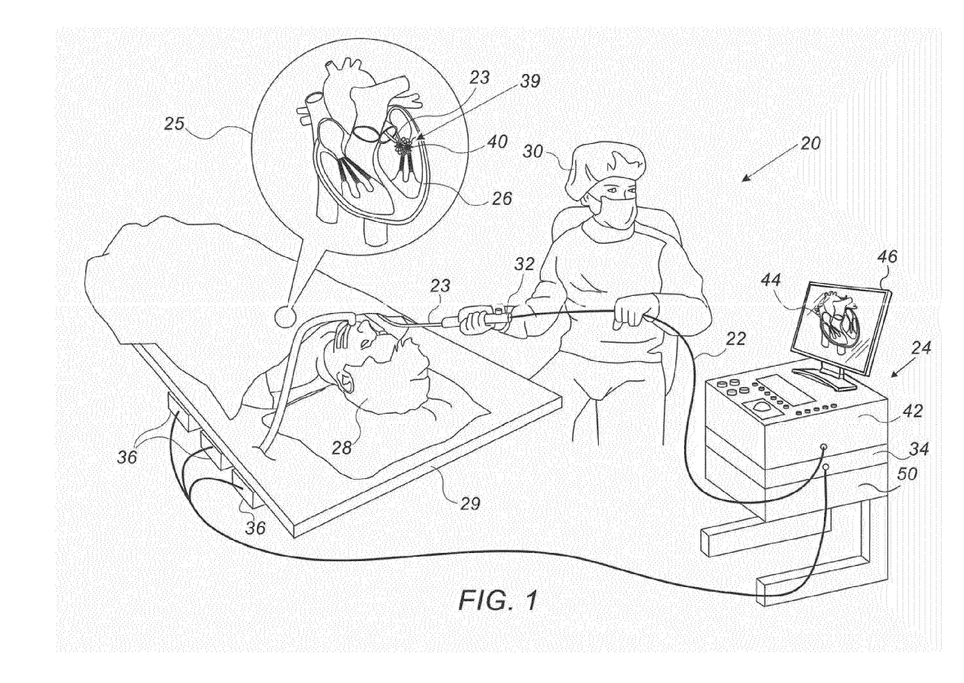

Fig. 1 is a schematic, pictorial illustration of a catheter-based position-tracking and ablation system

The system 20 comprises a catheter 22, in the present example an expandable cardiac catheter having a basket shape, and a control console 24.

The catheter 22 may be used for any suitable therapeutic and/or diagnostic purposes, such as but not limited to sensing of electrocardiograms (ECGs) in tissue in question and applying ablation signals to tissue of a heart 26.

Console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory 50. The software may be downloaded to console 24 in electronic form, over a network, for example, or it may be provided on non-transitory tangible media, such as optical, magnetic, or electronic memory media. Alternatively, some or all of the functions of processor 42 may be carried out using an application-specific integrated circuit (ASIC) or any suitable type of programmable digital hardware components.

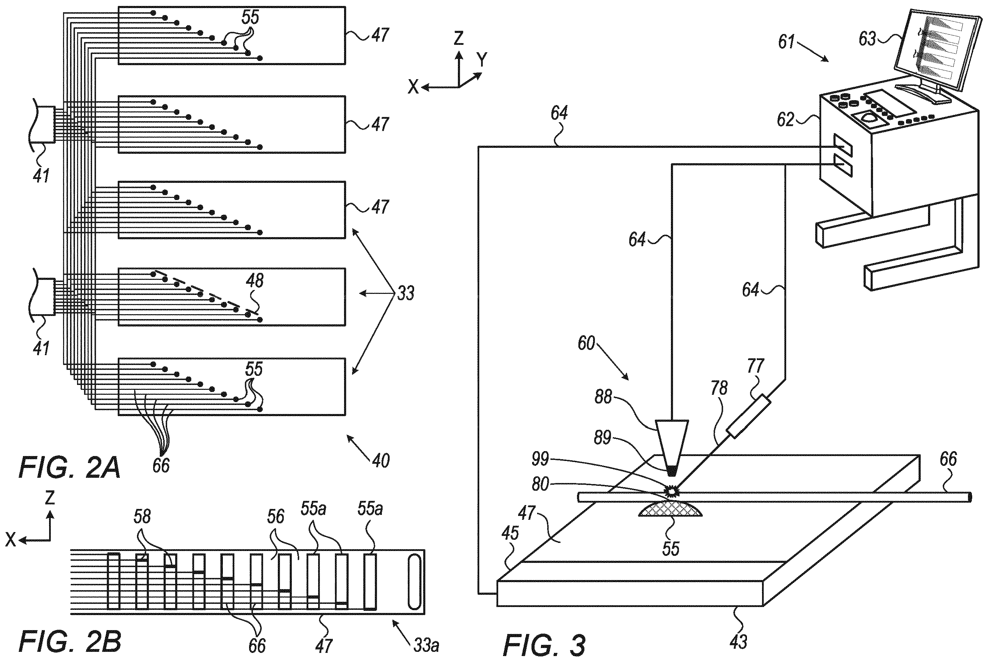

Reference is now made to an inset 25. In some examples, catheter 22 comprises a distal-end assembly 40 having multiple splines whose structure is shown in detail in Figs. 2A and 2B below, and a shaft 23 for inserting distal-end assembly 40 to a target location for ablating tissue in heart 26. During an EA sensing and/or an ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to interface circuitry of processor 42.

In some examples, catheter 22 comprises a position sensor 39 of a position tracking system, which is coupled to the distal end of catheter 22, e.g., in close proximity to distal-end assembly 40. In the present example, position sensor 39 comprises a magnetic position sensor, but in other examples, any other suitable type of position sensor (e.g., other than magnetic based) may be used.

Reference is now made back to the general view of Fig. 1. In some examples, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some examples, console 24 comprises a driver circuit 34, configured to drive magnetic field generators 36. Magnetic field generators 36 are placed at known positions external to patient 28, e.g., below table 29.

The processor 42 is configured to display, e.g., on a display 46 of console 24, the tracked position of distal-end assembly 40 overlaid on an image 44 of heart 26.

The method of position sensing using external magnetic fields is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

The example configuration shown in Fig. 1 is chosen by way of example for the sake of conceptual clarity. The techniques disclosed in Figs. 2-4 below may be applied, *mutatis mutandis,* using other components and settings of system 20.

### PRODUCING CONNECTIONS IN SPLINES OF A BASKET CATHETER

Fig. 2A is a schematic, pictorial illustration of splines 33 of distal-end assembly 40. Five splines 33 (also referred to herein as spines) are presented in the example of Fig. 2A, but distal-end assembly 40 may comprise any suitable number of splines 33, e.g., about ten splines.

In some examples, splines 33 are typically similar to one another, each spline 33 comprises a substrate 47 having solder pads, also referred to herein as pads 55 disposed thereon, and leads 66 configured to be attached (e.g., soldered) to pads 55.

In some examples, each lead 66 has a circular cross section having a diameter of about 32 µm, but in other examples lead 66 may have any other suitable shape of cross-section and respective size. Lead 66 is configured to conduct signals between (i) a respective pad 55 connected thereto, and (ii) manipulator 32 and/or console 24, via one or more shielded cables 41 of leads 66 or other suitable electrically conductive wires of catheter 22, such as a long flex circuit.

In the present examples, each spline 33 comprises about ten pads 55 disposed parallel to one another along the longitudinal axis of spline 33, which is parallel to an X-axis of an XYZ coordinate system. During the production of splines 33, each lead 66 is attached (e.g., soldered) to a respective pad 55 and the high density of pads 55 and leads 66 may cause an electrical short between adjacent pairs of pads 55 and leads 66 soldered with one another.

In some examples, pads 55 are arranged along a virtual diagonal line 48, so as to increase the distance between adjacent pads 55, and thereby, to prevent or reduce the occurrence of the electrical short described above. In other examples, the increased distance between adjacent pads 55 may be obtained using any other suitable arrangement of pads 55 on the surface of substrate 47.

Fig. 2B is a schematic, pictorial illustration of a spline 33a of distal-end assembly 40. In some examples, one or more splines 33a may replace one or more respective splines 33 of Fig. 2A above.

In some examples, spline 33a comprises a multi-layered structure formed on substrate 47 described in Fig. 2A above, or on any other suitable substrate. In the present example, spline 33a comprises solder pads 55a made from stripes of a flexible circuit board (flex CB) disposed parallel to the Z-axis of spline 33a.

In some examples, an electrically insulating layer 56 is disposed over substrate 47 and solder pads 55a, and leads 66 are disposed over layer 56. Moreover, spline 33a comprises slot vias 58, which are formed through layer 56, are filled with copper or with any other suitable electrically conductive substance, and are configured to conduct signals between solder pads 55a and leads 66. Note that in the example of Fig. 2B, substrate 47 appears in white color, and layer 56 is transparent for showing the structure of solder pads 55a and slot vias 58.

In the present example, slot vias 58 and solder pads 55a have approximately the same size along the X-axis of spline 33a. This configuration improves the mechanical strength of the connection between pads 55a and leads 66 of spline 33a.

### SYSTEM AND METHOD FOR SEMI-AUTOMATIC SOLDERING PROCESSES

Fig. 3 is a schematic, pictorial illustration of a system 60 for soldering leads 66 to respective soldering pads 55 disposed on substrate 47 of spline 33.

The system 60 comprises a fixture, referred to herein as a soldering fixture (SF) 45 also referred to herein as a fixture for brevity, which is configured to hold: (i) substrate 47 of distal-end assembly 40, and (ii) lead 66 placed (e.g., by an operator or a robotic arm, both not shown) on solder pad 55.

The system 60 comprises a laser assembly 77, which is configured to emit a laser beam 78, in the present example toward substrate 47. Note that in the present example laser beam 78 is used for marking a position of interest, and is not used for soldering lead 66 to pad 55.

The system 60 comprises a positioning assembly (PA) 43, also referred to herein as a mount or a stage, which is configured to move SF 45 along X, Y and Z axes of the XYZ coordinate system. In the present examples, PA 43 is configured to move SF 45 with substrate 47 and lead 66, relative to laser assembly 77, so as to mark a position 80, also referred to herein as a soldering position, at which lead 66 is to be attached (e.g., soldered) to pad 55, with a laser spot 99 of laser beam 78. In other words, lead 66 is positioned over pad 55, and spot 99 marks the soldering position therebetween.

In some examples, system 60 may comprise a camera (not shown), which is configured to acquire images of the surface of substrate 47. The system comprises a processor 62 (described in detail below), which is configured to display the image acquired by the camera on a display 63, which is described below.

The processor 62 is configured to identify position 80, e.g., by applying a pattern recognition software to the image acquired by the camera. The processor 62 is connected, via cables 64 or wirelessly to PA 43, laser assembly 77 and a soldering gun 88 (described below). Processor 62 is configured to control laser assembly 77 and PA 43 for positioning laser spot 99 on the surface of lead 66 at the identified position 80. Note that using the camera and processor 62 enables fully automatic positioning of laser spot 99 at position 80.

In such examples, PA 43 is configured to identify pad 55 among multiple pads 55 that are disposed on substrate 47 for conducting electrical signals between distal end assembly 40 and the proximal end of catheter 22. The identification of the specific pad 55 may be carried out using the pattern recognition software as described above. As an alternative, the substrate 47 can be masked with a specific pattern (e.g., a striped pattern or a green screen) so that the pads 55 can be recognized against the background pattern. Thereafter, the laser can be programmed to store the specific location of each of the pads 55 prior to applying the laser light for welding.

In other examples, at least part of the positioning of laser spot 99 may be carried out manually, e.g., without applying the pattern recognition software.

In some examples, system 60 comprises an operating console 61 having processor 62, display 63 and other components described herein. Console 61 may comprise a general-purpose computer, with suitable front end and interface circuits for receiving signals from and for controlling the components of system 60 as will be described herein. Processor 62 may be programmed in software to carry out the functions that are used by the system, and is configured to store data for the software in a memory of console 61. The software may be downloaded to console 61 in electronic form, over a network, for example, or it may be provided on non-transitory tangible media, such as optical, magnetic, or electronic memory media. Alternatively, some or all of the functions of processor 62 may be carried out using an application-specific integrated circuit (ASIC) or any suitable type of programmable digital hardware components.

The system 60 comprises soldering gun 88, which is configured to attached (e.g., solder) lead 66 to pad 55 at position 80. Soldering gun 88 is controlled by processor 62, and may comprise an ultrasound transducer 89, which is configured to ultrasonically bond lead 66 to pad 55 at position 80 by applying an ultrasound signal to lead 66 and pad 55 at position 80. In the context of the present disclosure and in the claims, the terms "ultrasonically bonded" and "ultrasonically attached" and their grammatical variations refer to ultrasonic techniques for welding or soldering between leads 66 and solder pads 55 of Fig. 2A above. In other examples, soldering gun 88 is configured to carry out the soldering process using any other suitable soldering and/or welding technique known in the art.

In some examples, positioning assembly 43 is configured to move at least soldering gun 88 relative to substrate 47 for aligning soldering gun 88 and laser spot 99 before applying the ultrasound signal.

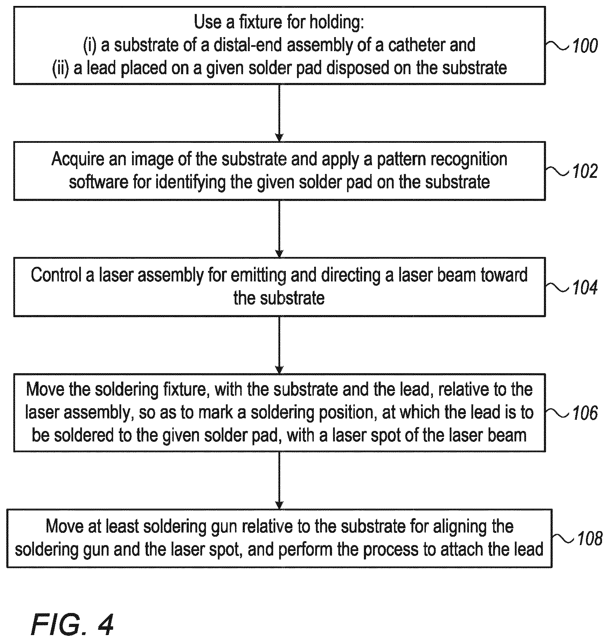

Fig. 4 is a flow chart that schematically illustrates a method for soldering lead 66 to pad 55 of spline

The method begins at a substrate and lead fixating step 100 with using SF 45 for holding: (i) substrate 47 of a respective spline 33 of distal-end assembly 40, and (ii) lead 66 placed on a given solder pad, e.g., pad 55 of Fig. 2A above, disposed on substrate 47, as described in detail in Fig. 3 above.

At a solder pad identification step 102, processor 62 receives an image of the surface of substrate 47 having multiple pads 55, and applied to the image a pattern recognition software configured to identify the position of (the given solder) pad 55 on substrate 47, as described in detail in Fig. 3 above.

At a laser beam directing step 104, processor 62 is configured to control laser assembly 77 for emitting and directing laser beam 78 toward substrate 47, as described in detail in Fig. 3 above.

At a fixture moving step 106, processor 62 is configured to control positioning assembly 43 to move soldering fixture 45 together with substrate 47 and lead 66, relative to laser assembly 77, so as to mark position 80, at which lead 66 is to be attached (e.g., soldered) to (the given solder) pad 55. In the present example, position 80 is marked using laser spot 99 of laser beam 78, as described in detail in Fig. 3 above.

At a soldering step 108 that concludes the method, processor 62 is configured to move the soldering gun 88 relative to substrate 47 for aligning soldering gun 88 and laser spot 99, and subsequently, perform the process to attach lead 66 to pad 55 by applying the ultrasound signal to lead 66 and pad 55 at position 80, as described in Fig. 3 above.

The method of Fig. 4 is simplified for the sake of conceptual clarity and is provided by way of example. In other examples, the order of the steps may alter for the sake of improved productivity and/or for any suitable considerations.

Although the examples described herein mainly address soldering of leads to soldering pads in basket catheters, the methods and systems described herein can also be used in other applications of soldering processes, such as in soldering leads to flex circuits which are then mounted onto balloons.

It will thus be appreciated that the examples described above are cited by way of example, and that the present invention is not limited to what has been particularly shown and described hereinabove. Rather, the scope of the present invention is defined by the appended claims.

## Claims

1. A system (60), comprising:
a processor (62);
a fixture (45), which is configured to hold (i) a substrate (47) of a distal-end assembly (40) of a catheter (22) and (ii) a lead (66) placed on a given solder pad (55) disposed on the substrate (47);
a laser assembly (77), which is controlled by the processor (62) to emit a laser beam (78);
a positioning assembly (43), which is controlled by the processor (62) to move, in use, the fixture (45), with the substrate (47) and the lead (66), relative to the laser assembly (77), so as to mark a soldering position (80), at which the lead (66) is to be attached to the given solder pad (55), with a laser spot (99) of the laser beam (78); and
a soldering gun (88), which is controlled by the processor (62) to attach the lead to the given soldering pad at the soldering position.

2. The system according to claim 1, wherein the processor (62) is further configured to: (i) identify the given solder pad among multiple solder pads that are disposed on the substrate for conducting electrical signals between a distal end and a proximal end of the catheter, and (ii) control the positioning assembly to move the fixture for marking the soldering position.

3. The system according to claim 1 or claim 2, wherein the positioning assembly (43) is configured to move at least the soldering gun (88) relative to the substrate for aligning the soldering gun and the laser spot.

4. The system according to claim 1 or claim 2, wherein the soldering gun (88) comprises an ultrasound transducer, which is configured to ultrasonically attach the lead (66) to the given soldering pad (55) at the soldering position by applying an ultrasound signal to the soldering position.

5. A method, comprising:
holding: (i) a substrate (47) of a distal-end assembly (40) of a catheter (22) and (ii) a lead (66) placed on a given solder pad (55) disposed on the substrate (47);
emitting a laser beam (78) toward the substrate (47);
moving the substrate (47) and the lead (66), relative to the laser beam (78), so as to mark a soldering position (80), at which the lead (66) is to be attached to the given solder pad (55), with a laser spot (99) of the laser beam (78); and
attaching by soldering the lead (66) to the given soldering pad (55) at the soldering position (80).

6. The method according to claim 5, wherein moving the substrate (47) and the lead (66) comprises: (i) identifying the given solder pad among multiple solder pads that are disposed on the substrate for conducting electrical signals between a distal end and a proximal end of the catheter, and (ii) moving the substrate for marking the soldering position.

7. The method according to claim 5 or claim 6, wherein attaching the lead (66) to the given soldering pad (55) comprises moving at least a soldering gun that carries out the attaching relative to the substrate for aligning the soldering gun and the laser spot

8. The method according to claim 5 or claim 6, wherein attaching the lead (66) to the given soldering pad (55) comprises applying an ultrasound signal to the soldering position.

## Patentansprüche

1. System (60), umfassend:
einen Prozessor (62);
eine Halterung (45), die konfiguriert ist, um (i) ein Substrat (47) einer Baugruppe (40) am distalen Ende eines Katheters (22) und (ii) ein Lot (66) zu halten, das auf einem gegebenen Lötpad (55) angeordnet ist, welches sich auf dem Substrat (47) befindet;
eine Lasereinheit (77), die vom Prozessor (62) gesteuert wird, um einen Laserstrahl (78) abzugeben;
eine Positionierungsvorrichtung (43), die vom Prozessor (62) gesteuert wird, um im Betrieb die Halterung (45) mit dem Substrat (47) und dem Lot (66) relativ zur Lasereinheit (77) zu bewegen, um eine Lötposition (80), an der das Lot (66) an dem gegebenen Lötpad (55) befestigt werden soll, mit einem Laserspot (99) des Laserstrahls (78) zu markieren; und
einen Lötkolben (88), der von dem Prozessor (62) gesteuert wird, um das Lot an der Lötposition an dem gegebenen Lötpad anzubringen.

2. System nach Anspruch 1, wobei der Prozessor (62) ferner konfiguriert ist um: (i) das gegebene Lötpad unter mehreren Lötpads zu identifizieren, die auf dem Substrat angeordnet sind, um elektrische Signale zwischen einem distalen Ende und einem proximalen Ende des Katheters zu leiten, und (ii) die Positionierungsvorrichtung zu steuern, um die Halterung zum Markieren der Lötposition zu bewegen.

3. System nach Anspruch 1 oder Anspruch 2, wobei die Positionierungsvorrichtung (43) konfiguriert ist, um mindestens den Lötkolben (88) relativ zum Substrat zu bewegen, um den Lötkolben und den Laserspot auszurichten.

4. System nach Anspruch 1 oder Anspruch 2, wobei der Lötkolben (88) einen Ultraschallwandler umfasst, der konfiguriert ist, um das Lot durch Anlegen eines Ultraschallsignals an die Lötposition ultraschalltechnisch an der Lötposition an dem gegebenen Lötpad (55) anzubringen.

5. Verfahren, umfassend:
Halten: (i) eines Substrats (47) einer Vorrichtung am distalen Ende (40) eines Katheters (22) und (ii) eines Lots (66), das auf einem gegebenen Lötpad (55) angeordnet ist, das sich auf dem Substrat (47) befindet;
Emittieren eines Laserstrahls (78) in Richtung des Substrats (47);
Bewegen des Substrats (47) und des Lots (66) relativ zum Laserstrahl (78), um eine Lötposition (80), an der das Lot (66) an dem gegebenen Lötpad (55) befestigt werden soll, mit einem Laserspot (99) des Laserstrahls (78) zu markieren; und
Befestigen des Lots (66) durch Löten an dem gegebenen Lötpad (55) an der Lötposition (80).

6. Verfahren nach Anspruch 5, wobei das Bewegen des Substrats (47) und des Lots (66) umfasst: (i) Identifizieren des gegebenen Lötpads unter mehreren Lötpads, die auf dem Substrat angeordnet sind, um elektrische Signale zwischen einem distalen Ende und einem proximalen Ende des Katheters zu leiten, und (ii) Bewegen des Substrats zum Markieren der Lötposition.

7. Verfahren nach Anspruch 5 oder Anspruch 6, wobei das Anbringen des Lots (66) an dem gegebenen Lötpad (55) das Bewegen mindestens eines Lötkolbens, der das Befestigen relativ zum Substrat ausführt, zum Ausrichten des Lötkolbens und des Laserstrahls umfasst.

8. Verfahren nach Anspruch 5 oder Anspruch 6, wobei das Anbringen des Lots (66) an dem gegebenen Lötpad (55) das Anlegen eines Ultraschallsignals an die Lötposition umfasst.

## Revendications

1. Système (60), comprenant :
un processeur (62) ;
un dispositif (45), qui est conçu pour contenir (i) un substrat (47) d'un ensemble extrémité distale (40) d'un cathéter (22) et (ii) un fil (66) placé sur une pastille de soudure (55) donnée disposée sur le substrat (47) ;
un ensemble laser (77), qui est commandé par le processeur (62) pour émettre un faisceau laser (78) ;
un ensemble de positionnement (43), qui est commandé par le processeur (62) pour déplacer, en cours d'utilisation, le dispositif (45), avec le substrat (47) et le fil (66), par rapport à l'ensemble laser (77), de manière à marquer une position de soudure (80), au niveau de laquelle le fil (66) doit être fixé à la pastille de soudure (55) donnée, avec un point laser (99) du faisceau laser (78) ; et
un pistolet à souder (88), qui est commandé par le processeur (62) pour fixer le fil à la pastille de soudure donnée au niveau de la position de soudure.

2. Système selon la revendication 1, dans lequel le processeur (62) est en outre conçu pour : (i) identifier la pastille de soudure donnée parmi de multiples pastilles de soudure disposées sur le substrat pour conduire des signaux électriques entre une extrémité distale et une extrémité proximale du cathéter, et (ii) commander l'ensemble de positionnement pour déplacer le dispositif pour marquer la position de soudure.

3. Système selon la revendication 1 ou la revendication 2, dans lequel l'ensemble de positionnement (43) est conçu pour déplacer au moins le pistolet à souder (88) par rapport au substrat pour aligner le pistolet à souder et le point laser.

4. Système selon la revendication 1 ou la revendication 2, dans lequel le pistolet à souder (88) comprend un transducteur à ultrasons, qui est conçu pour fixer par ultrasons le fil à la pastille de soudure donné (55) au niveau de la position de soudage en appliquant un signal à ultrasons à la position de soudage.

5. Procédé, comprenant :
le maintien : (i) d'un substrat (47) d'un ensemble d'extrémité distale (40) d'un cathéter (22) et (ii) d'un fil (66) placé sur une pastille de soudure (55) donnée disposée sur le substrat (47) ;
l'émission d'un faisceau laser (78) en direction du substrat (47) ;
le déplacement du substrat (47) et du fil (66), par rapport au faisceau laser (78), de manière à marquer une position de soudure (80), au niveau de laquelle le fil (66) doit être fixé à la pastille de soudure (55) donnée, avec un point laser (99) du faisceau laser (78) ; et
fixer par soudure le fil (66) à la pastille de soudure (55) donnée au niveau de la position de soudage (80).

6. Procédé selon la revendication 5, dans lequel le déplacement du substrat (47) et du fil (66) comprend : (i) l'identification de la pastille de soudure donnée parmi de multiples pastilles de soudure disposées sur le substrat pour conduire des signaux électriques entre une extrémité distale et une extrémité proximale du cathéter, et (ii) déplacer le substrat pour marquer la position de soudure.

7. Procédé selon la revendication 5 ou la revendication 6, dans lequel la fixation du fil (66) à la pastille de soudure donnée (55) comprend le déplacement d'au moins un pistolet à souder qui effectue la fixation par rapport au substrat pour aligner le pistolet à souder et le point laser

8. Procédé selon la revendication 5 ou la revendication 6, dans lequel la fixation du fil (66) à la pastille de soudure donnée (55) comprend l'application d'un signal ultrasonore à la position de soudage.
